# EUROPÄISCHE PATENTSCHRIFT

(11) **EP 0 459 140 B1**
(45) Veröffentlichungstag und Bekanntmachung des Hinweises auf die Patenterteilung: **11.09.1996**
(21) Anmeldenummer: 91106568.8
(22) Anmeldetag: 24.04.1991
(51) Int. Cl.: C07D 215/48, C07D 215/54, C07D 401/12, C07D 215/56, C07D 401/04, A01N 25/32

(54) **Cyanochinoline als Herbizide**
Cyanoquinolines as herbicides
Cyanoquinolines comme herbicides

(30) Priorität: 03.05.1990 DE 4014171
(43) Veröffentlichungstag der Anmeldung: 04.12.1991
(73) Patentinhaber: BASF Aktiengesellschaft, 67063 Ludwigshafen (DE)
(72) Erfinder: Hagen, Helmut, Dr., W-6710 Frankenthal (DE); Pfister, Juergen, Dr., W-6720 Speyer (DE); Brill, Gunter, Dr., W-6733 Hassloch (DE); Nilz, Gerhard, Dr., W-6701 Dannstadt-Schauernheim (DE); Wuerzer, Bruno, Dr., W-6701 Otterstadt (DE); Westphalen, Karl-Otto, Dr., W-6720 Speyer (DE)

(56) Entgegenhaltungen:
- EP-A- 0 086 750
- EP-A- 0 090 258
- EP-A- 0 120 484
- EP-A- 0 126 487
- EP-A- 0 159 290
- EP-A- 0 252 350
- EP-A- 0 258 184
- EP-A- 0 308 897
- EP-A- 0 318 225
- EP-A- 0 354 994
- EP-A- 0 356 971
- EP-A- 0 368 212
- EP-A- 0 387 582
- WO-A-84/01947
- DE-A- 1 913 466
- DE-A- 2 363 459
- DE-B- 1 170 955
- GB-A- 1 398 066
- GB-A- 1 467 225
- LU-A- 65 139
- US-A- 2 507 146
- US-A- 2 991 285
- US-A- 4 362 876
- US-A- 4 497 651
- US-A- 4 540 786
- US-A- 4 929 128
- J.ORG CHEM, 1953, No 18, N0 12, pages 1755-1761
- J.CHEM SOC, 1969, No 5, pages 713-715
- CHEM PHARM BULL, 1969, No 17 (1), pages 140-144
- CHEM PHARM BULL, 1972, N0 20 (7), pages 1544-1550
- CHEM PHARM BULL. 1985, N0 33, (4), pages 1360-1366
- ARCH.PHARM, 1988, No 321 (12), pages 891-896
- IND.J.CHEM, SECT B, 1989, N0 28B (7), pages 562-573
- GAZZ.CHIM.ITAL, 1989, No 119 (12) pages 637-641
- SYNTH.COMMUN, 1990, No 20 (3), pages 469-475

## Beschreibung

Die vorliegende Erfindung betrifft herzizide Mittel, welche mindestens eine Cyanochinolinverbindung der allgemeinen Formel Ia oder Ib 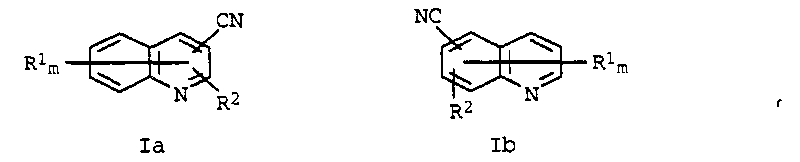 in der die Substituenten und der Index die folgende Bedeutung haben:
- R¹: Cyano, Halogen, C₁-C₄-Alkyl, C₁-C₄-Alkoxy oder Phenoxy, wobei der Phenylring ein bis drei der folgenden Reste tragen kann: Halogen, C₁-C₄-Alkyl, C₁-C₄-Halogenalkyl, C₁-C₄-Alkoxy, C₁-C₄-Alkylthio, C₂-C₄-Alkenyl, Nitro, Amino, Nitro-C₂-C₄-alkenyl und/oder Benzyl;
- m: 0, 1, 2 oder 3, wobei die Reste R¹ verschieden sein können, wenn m 2 oder 3 bedeutet;
- R²: Wasserstoff, Halogen oder eine Gruppe NR³R⁴;
- R³: Wasserstoff oder eine C₁-C₄-Alkylgruppe, welche ein bis fünf Halogenatome und/oder eine der folgenden Gruppen tragen kann: Hydroxy, C₁-C₄-Alkoxy, C₁-C₄-Halogenalkoxy, C₁-C₄-Alkylthio, Amino, C₁-C₄-Alkylamio oder Di-C₁-C₄-alkylamio;
- R⁴: eine der bei R³ genannten Gruppen; eine C₁-C₄-Alkylamiogruppe; eine Di-C₁-C₄-alkylamiogruppe; eine C₁-C₄-Alkylgruppe, welche einen der folgenden Reste trägt: C₁-C₄-Alkoxycarbonyl, 1-Pyrrolidinyl oder 1-Imidazolyl;
oder
- R³: und R⁴ gemeinsam
eine C₄-C₅-Alkylenkette, welche durch ein Sauerstoff- oder ein Stickstoff-Glied unterbrochen sein kann, wobei das Stickstoffatom seinerseits eine C₁-C₄-Alkylgruppe oder eine Amino-C₁-C₄-alkylgruppe tragen kann
oder eine 1,3-Butadienylkette, welche ein oder zwei der bei R¹ genannten Reste oder eine Formylgruppe tragen kann,
sowie landwirtschaftlich brauchbare Salze der Verbindungen Ia und Ib, sofern diese Verbindungen einen basischen Stickstoffsubstituenten oder einen sauren Hydroxysubstituenten enthalten und mindestens einen herbiziden Wirkstoff aus der Gruppe der Cyclohexenonoximether der Formel X, 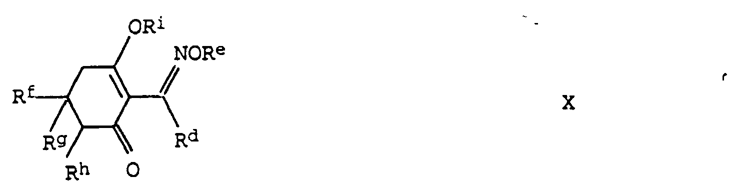 in welcher die Substituenten folgende Bedeutung haben:
- R^{d}: eine C₁-C₄-Alkylgruppe;
- R^{e}: eine C₁-C₄-Alkylgruppe, eine C₃-C₄-Alkenylgruppe, eine C₃-C₄-Alkinylgruppe, eine C₃-C₄-Halogenalkylengruppe oder eine Thenylgruppe, welche durch ein Halogenatom substituiert sein kann; eine C₃-C₄-Alkenylgruppe, welche einen Phenylrest trägt, wobei dieser ein bis drei der folgenden Reste tragen kann: Halogen, C₁-C₄-Alkyl, C₁-C₄-Akoxy, C₁-C₄-Halogenalkyl und/oder C₁-C₄-Halogenalkoxy;
- R^{f}: eine C₁-C₄-Alkylgruppe, welche ein- oder zweifach durch C₁-C₄-Alkylthio oder C₁-C₄-Alkoxy substituiert sein kann;
ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, und wobei dieser Ring bis zu drei der folgenden Reste tragen kann: Hydroxy, Halogen, C₁-C₄-Alkyl, C₁-C₄-Halogenalkyl, C₁-C₄-Alkoxy und/oder C₁-C₄-Alkylthio;
ein 10-gliedriger gesättigter oder einfach ungesättigter Heterocyclus, welcher zwei Sauerstoffatome oder Schwefelatome enthält und durch bis zu drei C₁-C₄-Alkylgruppen und/oder Methoxygruppen substituiert sein kann;
ein Phenylrest, ein Pyridylrest, ein Thiazolylrest, ein Pyrazolylrest, ein Pyrrolylrest oder ein Isoxazolylrest, wobei diese Reste bis zu drei der folgenden Gruppen tragen können: C₁-C₄-Alkyl, C₁-C₄-Halogenalkyl, C₁-C₄-Alkoxy, C₁-C₄-Alkylthio, C₃-C₆-Alkenyloxy, C₃-C₆-Alkinyloxy, C₁-C₄-Alkoxy-C₁-C₃-alkyl, C₁-C₄-Dialkoxy-C₁-C₃-alkyl, Formyl, Halogen und/oder Benzoylamino
- R^{g}: Wasserstoff, Hydroxy oder, wenn R^{f} die Bedeutung C₁-C₆-Alkylgruppe hat, eine C₁-C₆-Alkylgruppe;
- R^{h}: Wasserstoff, eine Cyanogruppe, ein Halogenatom, eine C₁-C₄-Alkoxycarbonylgruppe oder eine C₁-C₄-Alkylketoximgruppe und
- Rⁱ: Wasserstoff oder das Äquivalent eines umweltverträglichen Kations,
enthalten.

Eine Reihe von Cyanochinolinverbindungen sind aus der Literatur bekannt [z.B. Arch. Pharm. 321(12), 891 (1988); Chem. Heterocycl. Comp. Vol. 32/1, 180; Chem. Pharm. Bull. 17, 140 (1969); Chem. Pharm. Bull. 20, 1544 (1972); Chem. Pharm. Bull. 29, 1286 (1981); Chem. Pharm. Bull. 30, 851 (1982); Chem. Pharm. Bull. 33, 1360 (1985); Gazz. Chim. Ital. 119(12), 637 (1989); Ind. J. Chem. 28B(7), 562 (1989); J. Chem. Soc. C, 713 (1969); J. Org. Chem. 18(12), 1755 (1953); Synth. Commun. 20(3), 469 (1990); WO 84/01947; EP-A 086 750; EP-A 090 258; EP-A 120 484; EP-A 126 487; EP-A 252 350; EP-A 308 897; EP-A 318 225; EP-A 354 994 (Vergleichsbeispiel 39, Stufe 1); EP-A 356 971 (Bsp. 1, b): 2,3-Dicyanochinolin); US-A 2,507,146; US-A 2,991,285; US-A 4,362,876; US-A 4,497,651 (Bsp. 9); US-A 4,540,786; US-A 4,920,128; DE-A 11 70 955; DE-A 19 13 466; DE-A 23 17 230; DE-A 23 63 459; GB-A 1,398,066; GB-A 1,467,255].

Außerdem werden in der Literatur bestimmte Naphthalin-, Chinolin- oder 1,8-Naphthyridine als Antidots für Herbizide beschrieben [EP-A 159 290; EP-A 258 184; EP-A 368 212; EP-A 387 582].

Eine antidotisierende Wirkung von Cyanochinolinen zum Schutz von Kulturpflanzen vor Schädigungen, die durch die Anwendung von herbiziden Cyclohexenonderivaten hervorgerufen werden können, wird in der Literatur jedoch nicht beschrieben.

Cyclohexenonderivate werden in der Literatur als Herbizide beschrieben [EP-A 228 598; EP-A 230 235; EP-A 238 021; US-A 3,950,420; US-A 4,432,786]. Sie dienen vorwiegend zur Bekämpfung unerwünschter Gräser in dikotylen Kulturen und in Gräsern, die nicht zur Familie der Gramineen zählen. Je nach Struktur der Substituenten und Dosis der Anwendung können Verbindungen aus dieser Gruppe auch zur selektiven Bekämpfung von unerwünschten Gräsern in Gramineen Kulturen wie Weizen und Reis eingesetzt werden.

Der vorliegenden Erfindung lag nun die Aufgabe zugrunde, Verbindungen zu finden, welche die Nachteile, die bei der Verwendung der obengenannten Herbizide der Formel X bestehen, zumindest so stark verringern, daß der Ernteertrag der Kulturpflanzen nicht mehr oder nicht nennenswert herabgesetzt wird.

Demgemäß wurde gefunden, daß die eingangs definierten Verbindungen Ia und Ib bei getrennter oder gleichzeitiger Anwendung mit Herbiziden aus der Klasse der Cyclohexanone geeignet sind, Schädigungen von Kulturpflanzen zu unterdrücken.

Im Hinblick auf die bestimmungsgemäße Verwendung der Verbindungen Ia und Ib kommen als Substituenten bevorzugt folgende Rest in Betracht:
- R¹: Cyano; Halogen wie Fluor, Chlor, Brom und Jod, insbesondere Chlor und Brom;
Alkyl wie Methyl, Ethyl, Propyl, 1-Methylethyl, Butyl, 1-Methylpropyl, 2-Methylpropyl und 1,1-Dimethylethyl, insbesondere Methyl und Ethyl;
Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy und Ethoxy;
oder Phenoxy, welches ein bis drei der folgenden Reste tragen kann: Alkyl wie vorstehend genannt, insbesondere Methyl; Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Trifluormethyl; Alkoxy wie vorstehend genannt, insbesondere Methoxy, Ethoxy und 1-Methylethocy; Alkylthio wie Methylthio, EThylthio, Propylthio, 1-Methylethylthio, Butylthio, 1-Methylpropylthio, 2-Methylpropylthio und 1,1-Dimethylethylthio, insbesondere Methylthio; Alkenyl wie insbesondere Ethenyl, 1-Propenyl, 2-Propenyl, 1-Butenyl, 2-Butenyl, 3-Butenyl, 1-Methyl-1-propenyl, 2-Methyl-1-propenyl, 1-Methyl-2-propenyl und 2-Methyl-2-propenyl; Nitro, Amino, Nitrialkenyl wie insbesondere 2-Nitro-ethenyl und/oder Benzyl;
- m: 0, 1, 2 oder 3, wobei die Reste R¹ verschieden sein können, wenn m 2 oder 3 bedeutet;
- R²: Wasserstoff;
Halogen wie bei R¹ genannt, insbesondere Chlor und Brom;
oder eine Gruppe -NR³R⁴;
- R³: Wasserstoff;
Alkyl wie bei R¹ genannt, insbesondere Methyl und Ethyl, welche ein bis fünf der im allgemeinen und im besonderen bei R¹ genannten Halogenatome und/oder eine der folgenden Gruppen tragen kann: Hydroxy, Alkoxy wie bei R¹ genannt, insbesondere Fluor und Chlor;
Halogenalkoxy wie bei R¹ genannt, insbesondere 2,2,2-Trifluorethoxy;
Alkylthio wie bei R¹ genannt, insbesondere Methylthio und Butylthio;
oder Amino, welches ein oder zwei der bei R¹ genannten Alkylreste tragen kann, wobei diese Alkylreste bei zweifach substituiertem Amino verschieden sein können, insbesondere Dimethylamino;
- R⁴: eine der bei R³ im allgemeinen und im besonderen genannten Gruppen;
Amino, welches ein oder zwei der bei R¹ genannten Alkylreste trägt, wobei diese Alkylreste bei zweifach substituiertem Amino verschieden sein können, insbesondere Dimethylamino;
Alkyl wie-bei R¹ genannt, insbesondere Methyl und Ethyl;
welches einen der folgenden Reste trägt: Alkoxycarbonyl wie Methoxycarbonyl, Ethoxycarbonyl, Propyoxycarbonyl, 1-Methylethoxycarbonyl, Butyloxycarbonyl, 1-Methylpropyloxycarbonyl, 2-Methylpropyloxycarbonyl und 1,1-Dimethylethoxycarbonyl, insbesondere Methoxycarbonyl; 1-Pyrrolidinyl oder 1-Imidazolyl;
oder
R³ und R⁴ gemeinsam
eine Butylen- oder Pentylenkette, welche durch ein Stickstoffatom unterbrochen sein kann, wie -CH₂-CH₂-NH-CH₂CH₂- oder -CH2CH2-NH-CH2CH2CH2-, wobei das Stickstoffatom seinerseits eine Alkylgruppe wie bei R¹ genannt tragen kann, insbesondere Methyl und Ethyl, wobei diese Alkylgruppe ihrerseits insbesondere in 2-Position eine Aminogruppe tragen kann;
eine 1,3-Butadienylgruppe, welche ein oder zwei der bei R¹ genannten Reste, insbesondere Methyl, oder eine Formylgruppe tragen kann,
sowie die landwirtschaftlich brauchbaren Salze der Verbindungen Ia und Ib, sofern diese Verbindungen einen basischen Stickstoffsubstituenten oder einen sauren Hydroxysubstituenten enthalten.

Als Salze der Verbindungen der Formel Ia und Ib kommen landwirtschaftlich brauchbare Salze, beispielsweise Alkalimetallsalze, insbesondere das Kalium- oder Natriumsalz, Erdalkalimetallsalze, insbesondere das Calcium-, Magnesium- oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoniumsalze in Betracht.

Spezielle Beispiele für Cyclohexenone der Formel X, deren Kulturpflanzenverträglichkeit durch Verbindungen der Formel Ia und Ib verbessert werden kann, sind in der folgenden Tabelle B aufgeführt.

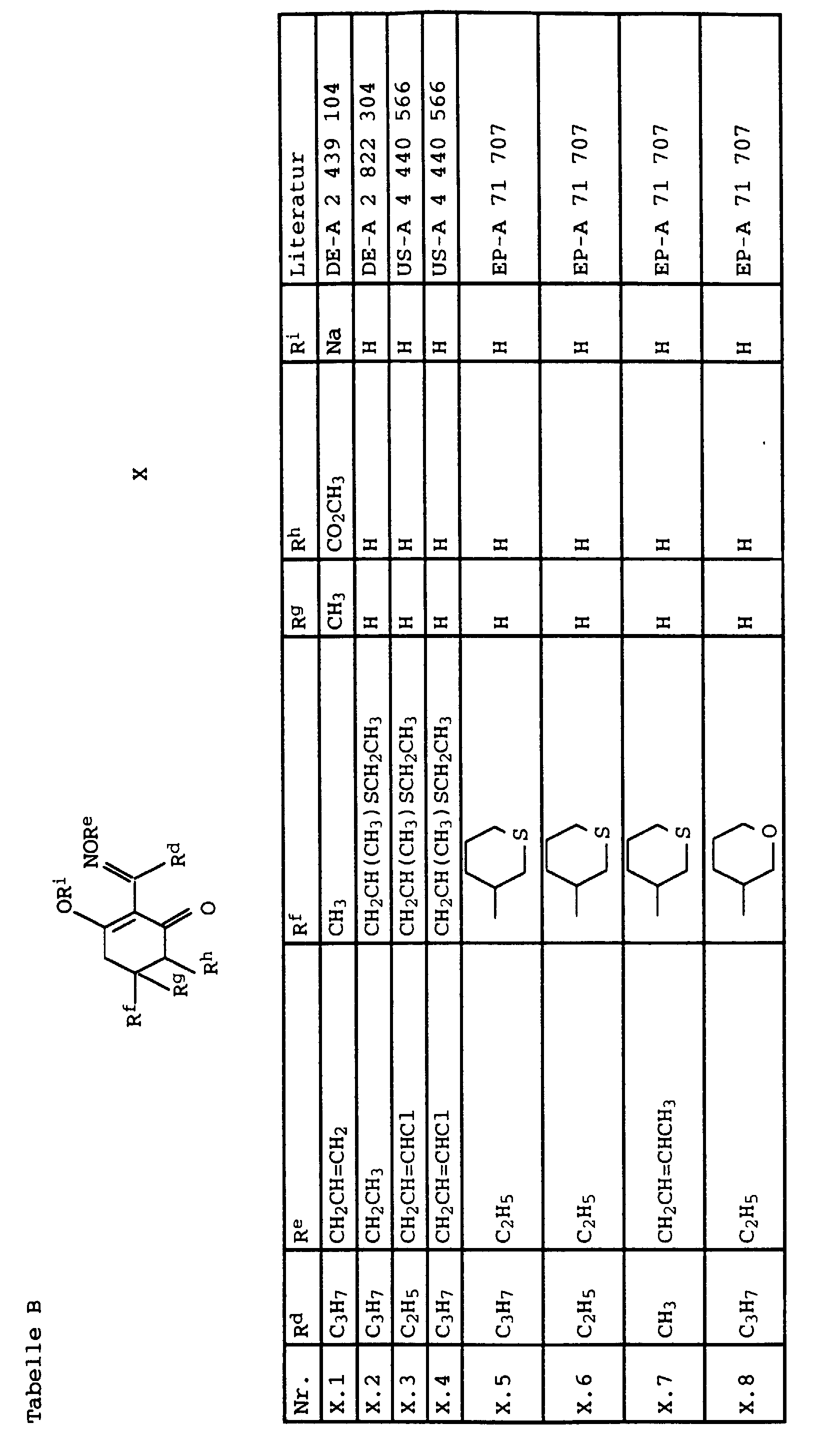

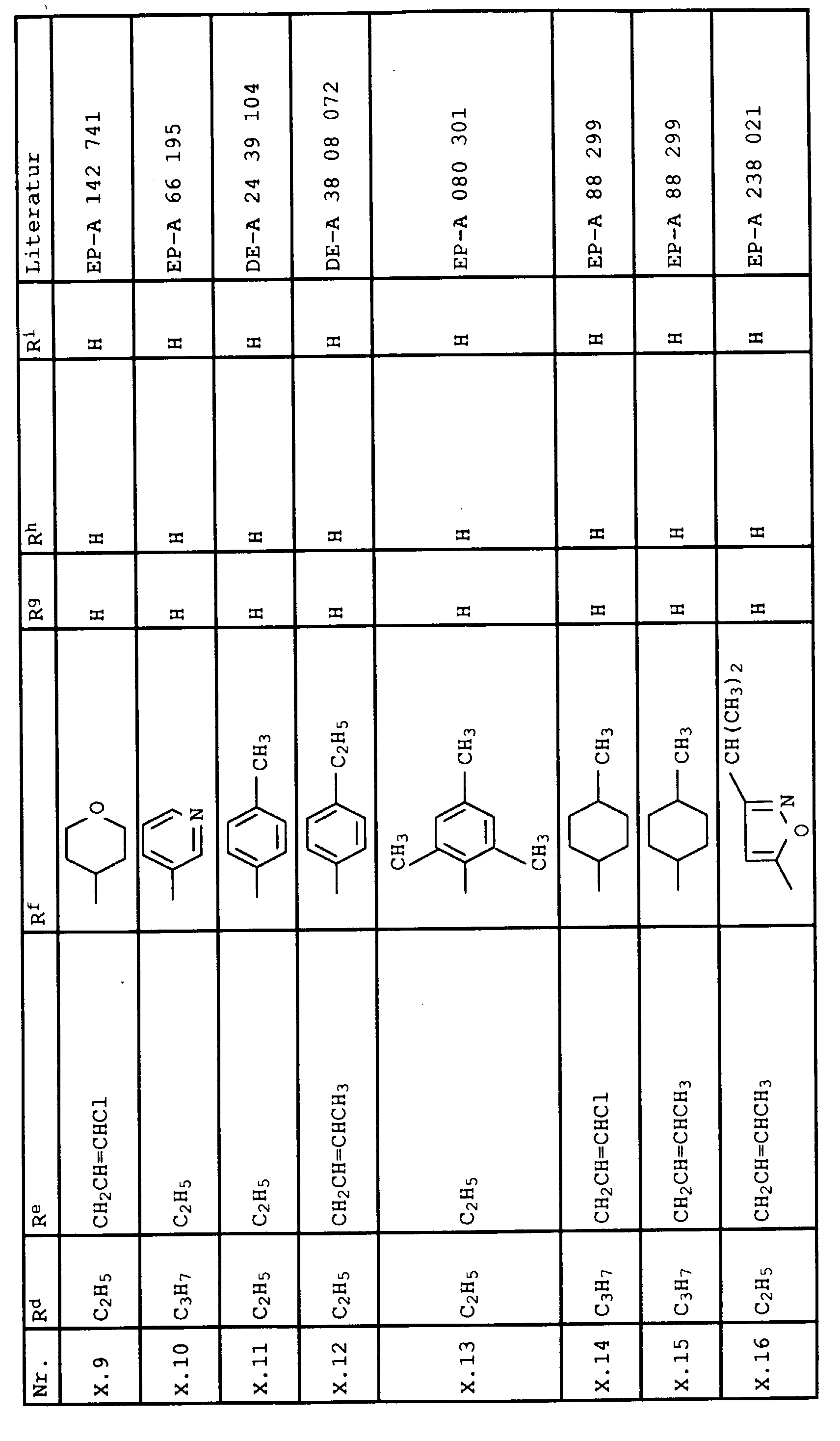

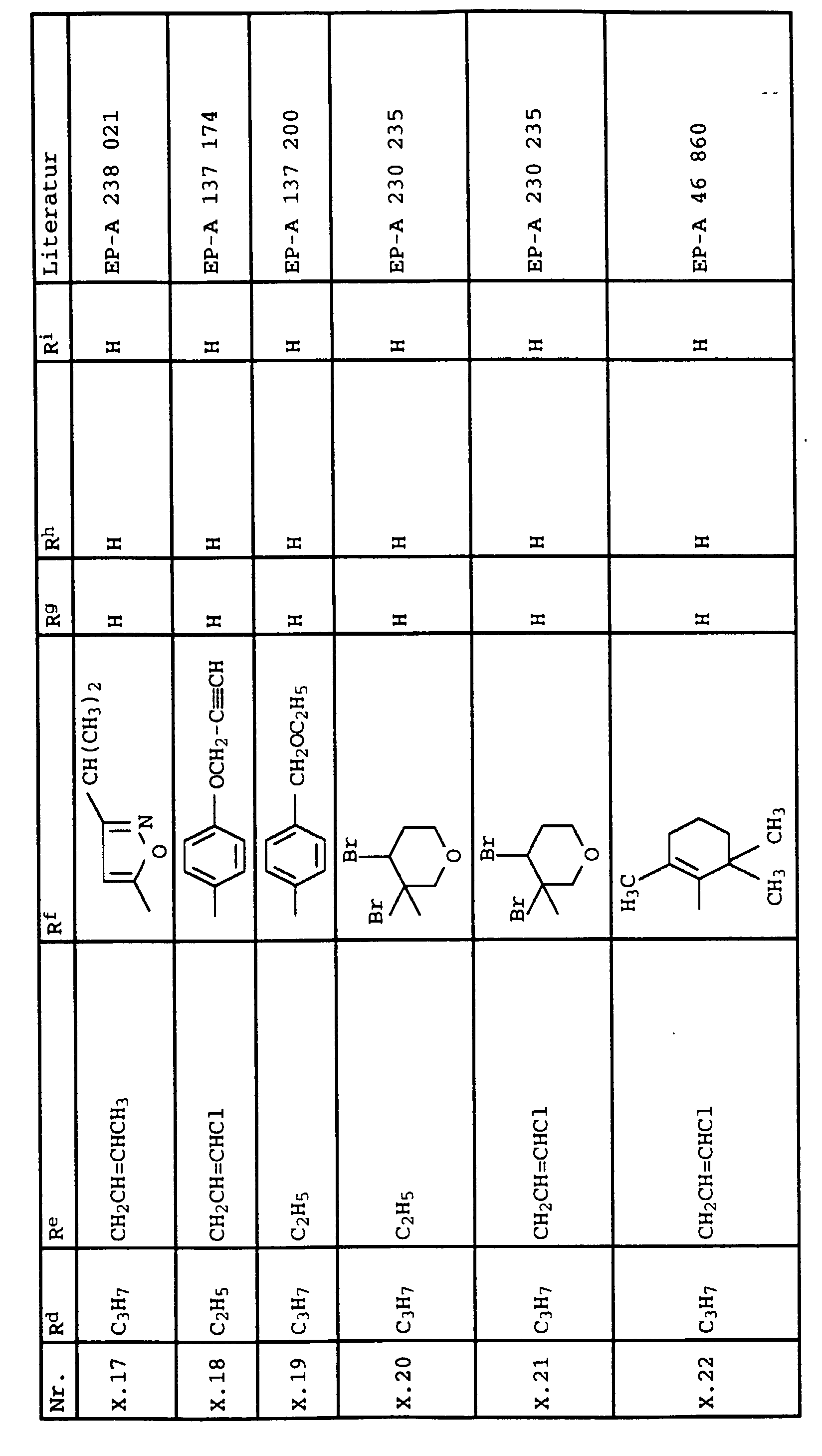

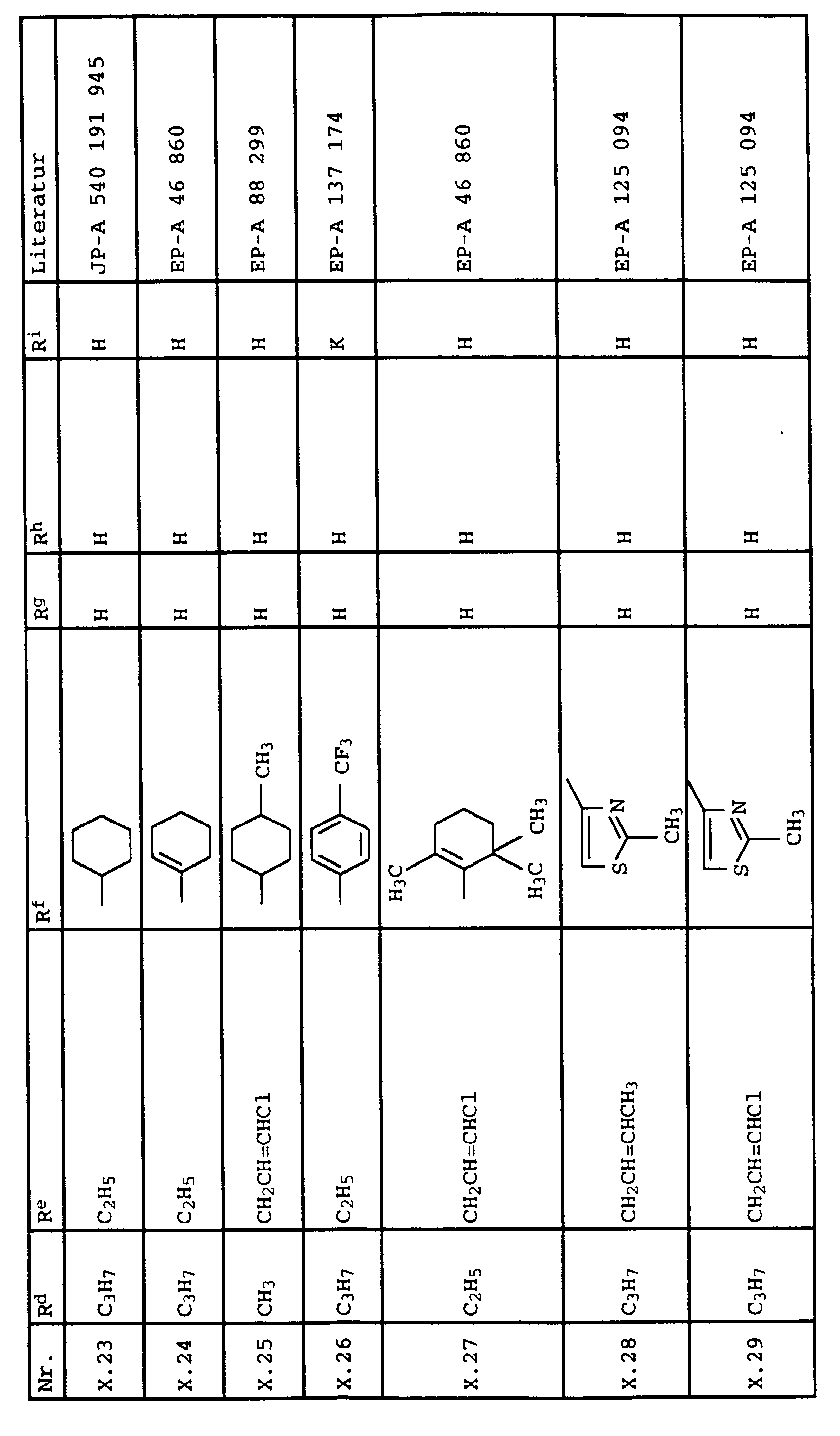

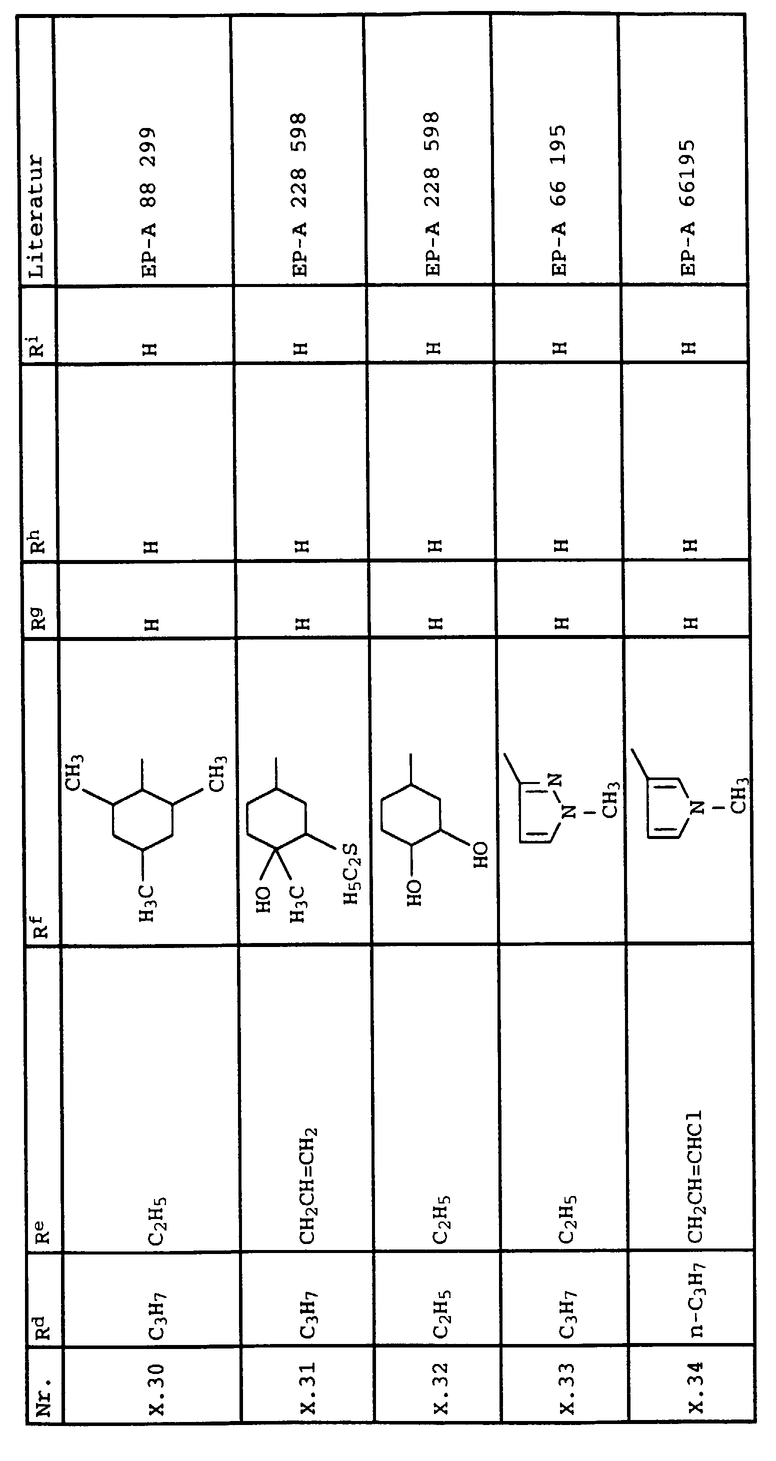

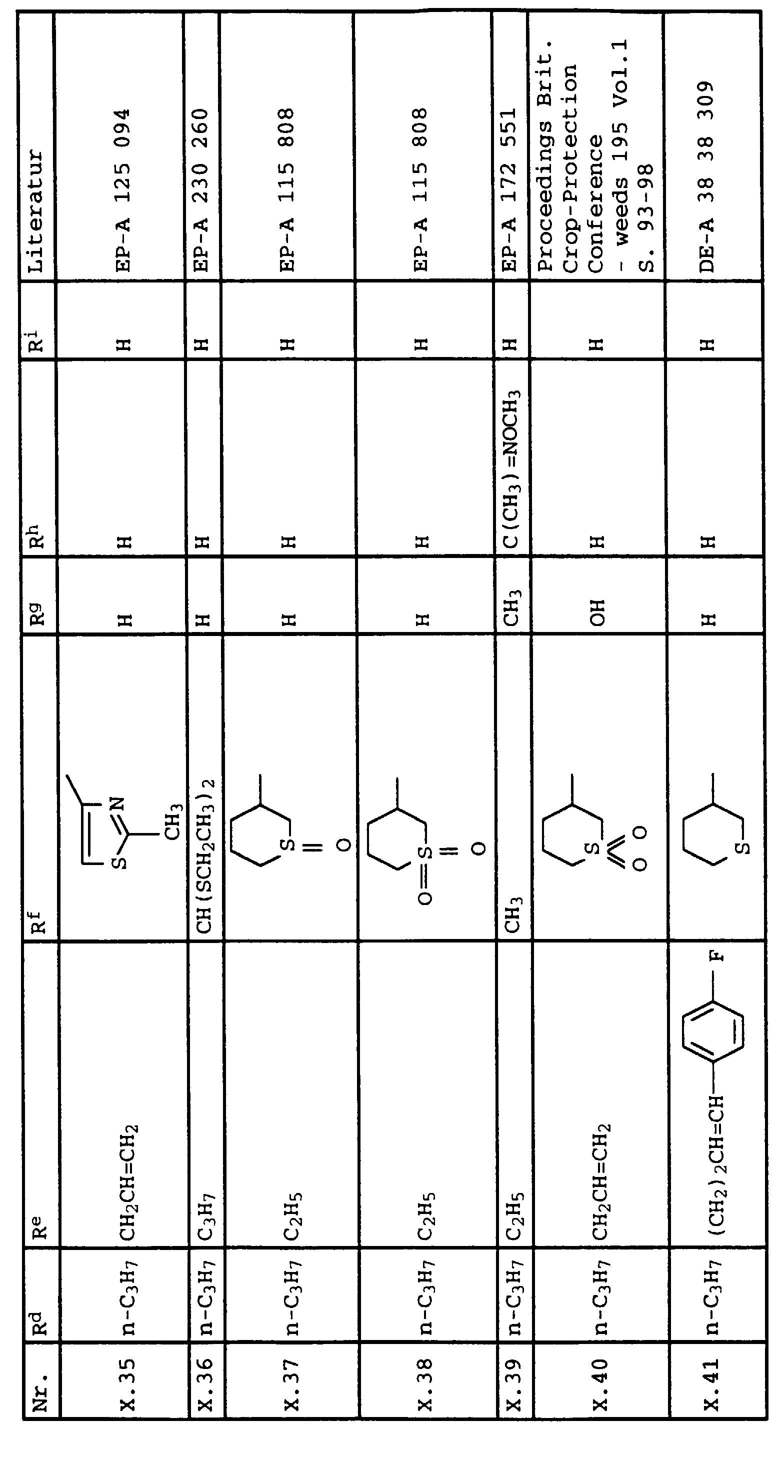

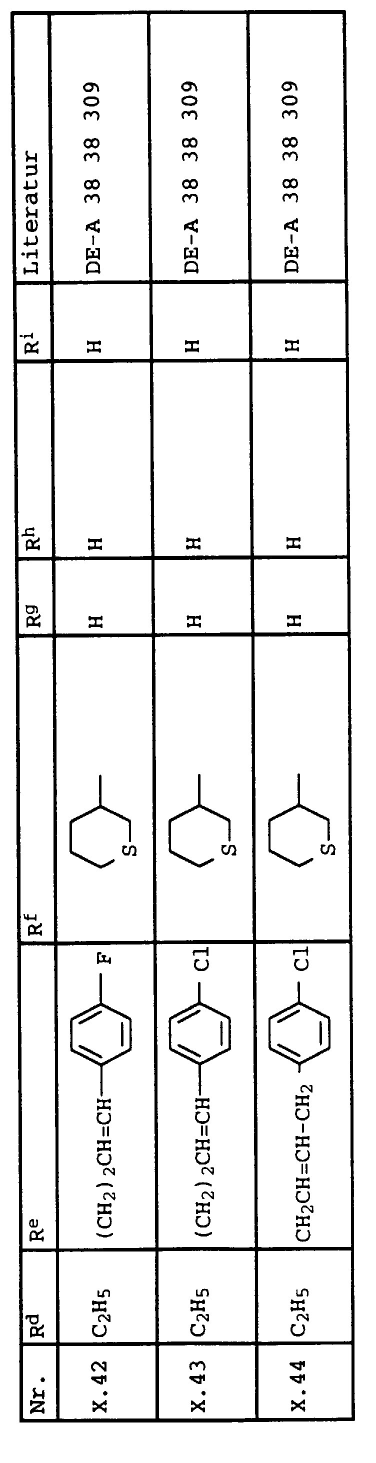

Herbizide Wirkstoffe und antidotisch wirkende Verbindungen können gemeinsam oder getrennt nach dem Auflaufen auf die Blätter und Sprosse der Kulturpflanzen und der unerwünschten Gräser ausgebracht werden. Bevorzugt wird das antidotisch wirkende Mittel gleichzeitig mit dem herbiziden Wirkstoff ausgebracht. Auch eine getrennte Ausbringung, wobei das Antidot zuerst und anschließend der herbizide Wirkstoff auf das Feld gebracht werden, ist möglich. Herbizider Wirkstoff und Antidot können hierbei als Spritzmittel in suspendierbarer, emulgierbarer oder löslicher Form gemeinsam oder getrennt formuliert vorliegen.

In der Praxis von Bedeutung ist auch eine Behandlung der Kulturpflanzensamen mit dem Antidot vor der Aussaat. Der herbizide Wirkstoff wird dann allein in der üblichen Weise appliziert.

Für das gleiche Cyclohexenonderivat X werden unterschiedliche Mengen einer antidotisch wirkenden Verbindung benötigt, wenn das Cyclohexenonderivat X in verschiedenen Kulturen eingesetzt wird. Die Mengenverhaltnisse, in denen ein Cyclohexenonderivat X und ein Cyanochinolin Ia oder Ib eingesetzt werden, sind in breiten Bereichen variabel. Sie sind unabhängig von der Struktur des Cyclohexenonderivats X, des Cyanochinolins Ia bzw. Ib und der jeweiligen Kultur. Geeignete Anteilverhältnisse herbizider Wirkstoff : antidotisch wirkender Verbindung liegen zwischen 1 : 10 bis 1 : 0,01: vorzugsweise 1 : 4 bis 1 : 0,25 Gew.-Teile sowohl bei gemeinsamer als auch bei getrennter Ausbringung.

Die neuen herbiziden Mittel können neben dem Cyanochinolin Ia bzw. Ib als Antidot und dem Herbizid aus der Gruppe der Cyclohexenone X weitere herbizide oder wachstumsregulierende Wirkstoffe anderer chemischer Struktur enthalten, wobei der antagonistische Effekt erhalten bleibt.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentige wäßrige, Ölige oder sonstige Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Ole pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon und Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot, Netz-, Haft-, Dispergier- oder Emulgiermittel und eventuell Lösungsmittel oder Öl Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenyl, Nonylphenol, Alkylphenylpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin-Sulfitablaugen und Methylcellulose in Betracht.

Pulver-, Streu- und Stäubmittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Kieselsäuren, Kieselgele, Silikate, Talkum, Kaolin, Attaclay, Kalkstein, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,1 und 95 Gew.-% an herbizidem Wirkstoff und Antidot, vorzugsweise zwischen 0,5 und 90 Gew.-%. Die Aufwandmengen an herbizidem Wirkstoff betragen 0,2 bis 5 kg/ha aktive Substanz (a.S.).

Die Herstellung der Verbindungen Ia und Ib wird durch die nach-' stehend beschriebenen Beispiele näher erläutert.

### Herstellungsbeispiele

### Beispiel 1

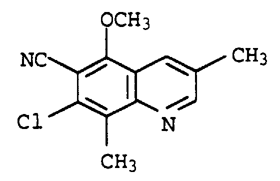

Eine Mischung aus 3,8 g (0,02 mol) 7-Chlor-3,8-dimethyl-5-nitrochinolin, 2,6 g (0,04 mol) Kaliumcyanid und 150 ml Methanol wurde 15 h unter Rückfluß zum Sieden erhitzt. Anschließend wurde die Reaktionsmischung mit Wasser versetzt und mit Methylenchlorid extrahiert. Aus der organischen Phase erhielt man nach chromatographischer Reinigung 1,5 g (38 %) des Produkts; Fp. 173-174°C; Wirkstoffbeispiel 4.001

### Beispiel 2

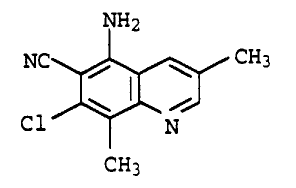

Eine Mischung aus 11,8 g (0,05 mol) 7-Chlor-3,8-dimethyl-5-nitrochinolin, 150 ml Dimethylformamid (wasserfrei), 16 ml (0,15 mol) Cyanessigsäureethylester, 5,5 g (0,10 mol) Kaliumhydroxid und 3,3 g (0,05 mol) Kaliumcyanid wurde 15 h bei 50°C belassen. Anschließend wurde das Lösungsmittel bei vermindertem Druck abdestilliert und der so erhaltene Rückstand wurde mit 100 ml Natronlauge (5 %ige wäßrige Lösung) 0,5 h bei 80°C gehalten. Beim Abkühlen fiel das Produkt als Feststoff aus.

Ausbeute 8,4 g (72 %); Fp.: 260-265°C; Wirkstoffbeispiel 4.002

### Beispiel 3

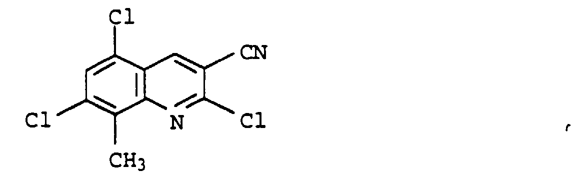

Eine Mischung aus 60 g (0,38 mol) Phosphonoxychlorid, 15 g (0,14 mol) Triethylamin und 33,5 g (0,15 mol) 7-Chlor-3-cyano-2,4-dihydroxy-8-methylchinolin wurde 6 h unter Rückfluß zum Sieden erhitzt. Nach Abkühlen und Hydrolyse der Reaktionsmischung mit 500 g Eis wurden 21 g (55 %) festes Produkt isoliert. Fp.: 135°C.

### Beispiel 4

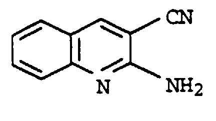

60,5 g (0,5 mol) o-Aminobenzaldehyd und 35 g (0,53 mol) Malondinitril wurden in 500 ml Ethanol mit 1 ml Pyridin 3 h zum Sieden erhitzt. Nach Abkühlen fielen 72,5 g (86 %) des festen Produktes aus. Fp.: 235°C.

Lit: E.C. Taylor, N.W. Kalenda, J. Org. Chem. 18 (1953), 1755

Die in den nachstehenden Tabellen aufgeführten Wirkstoffe können analog zu diesen Verfahrensbeispielen hergestellt werden.

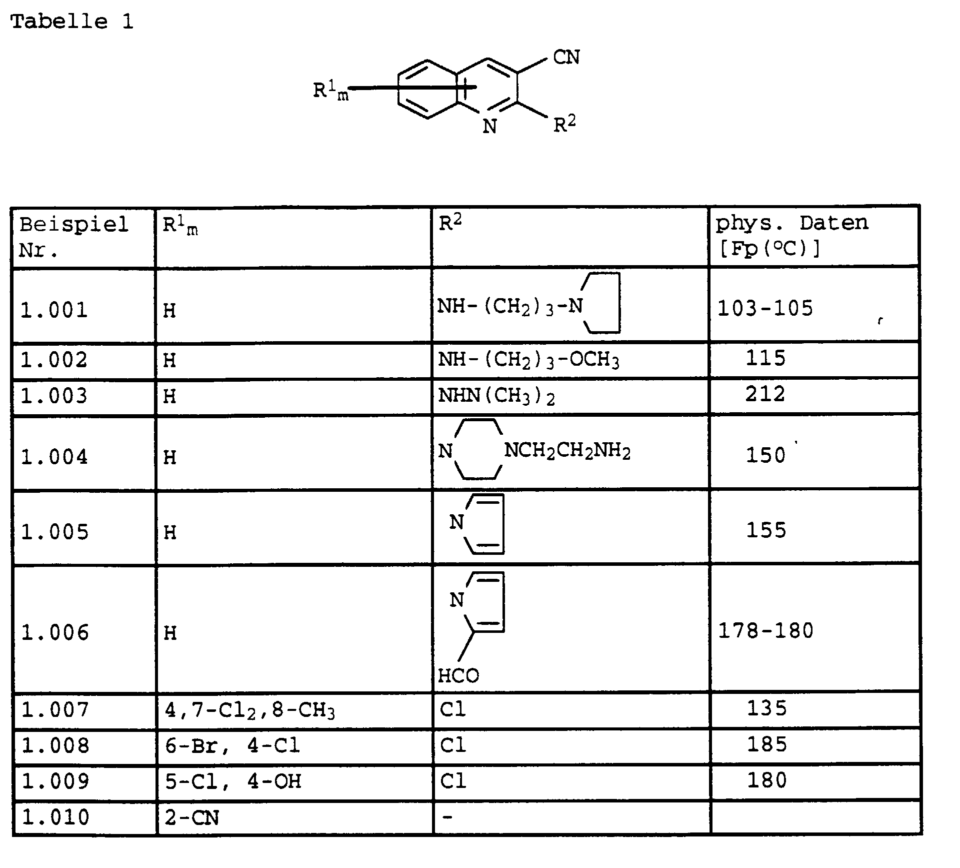

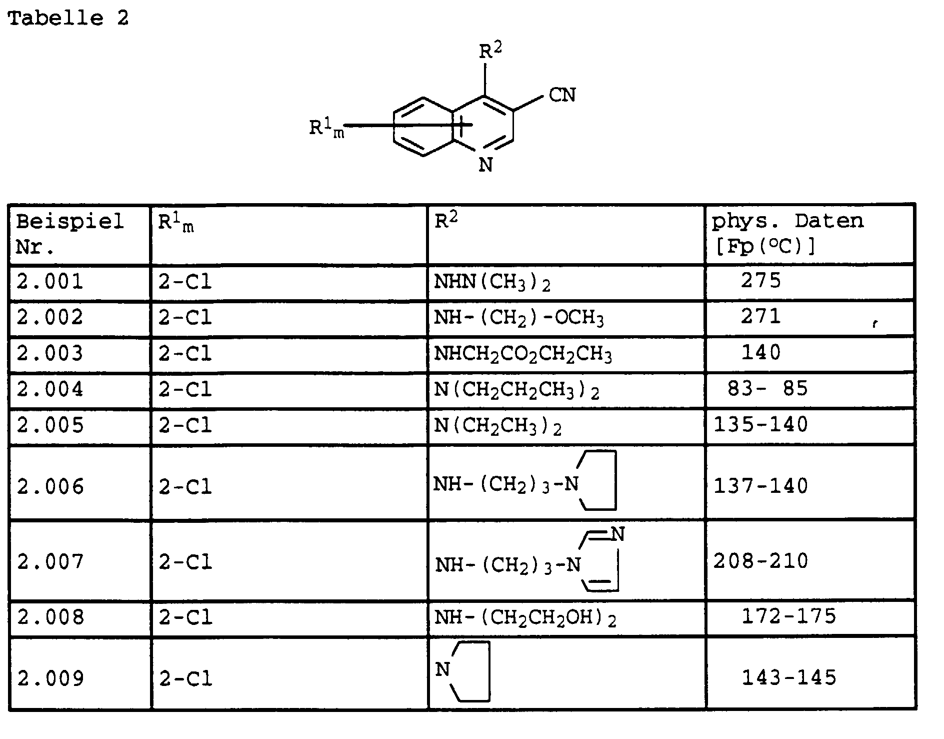

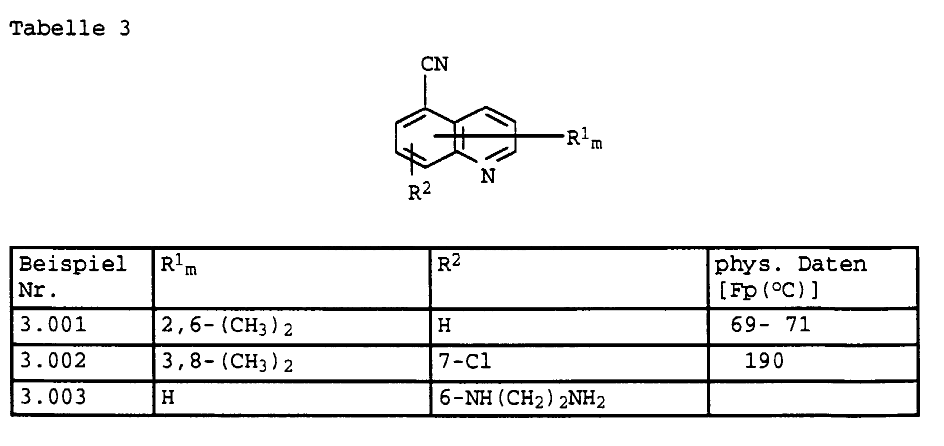

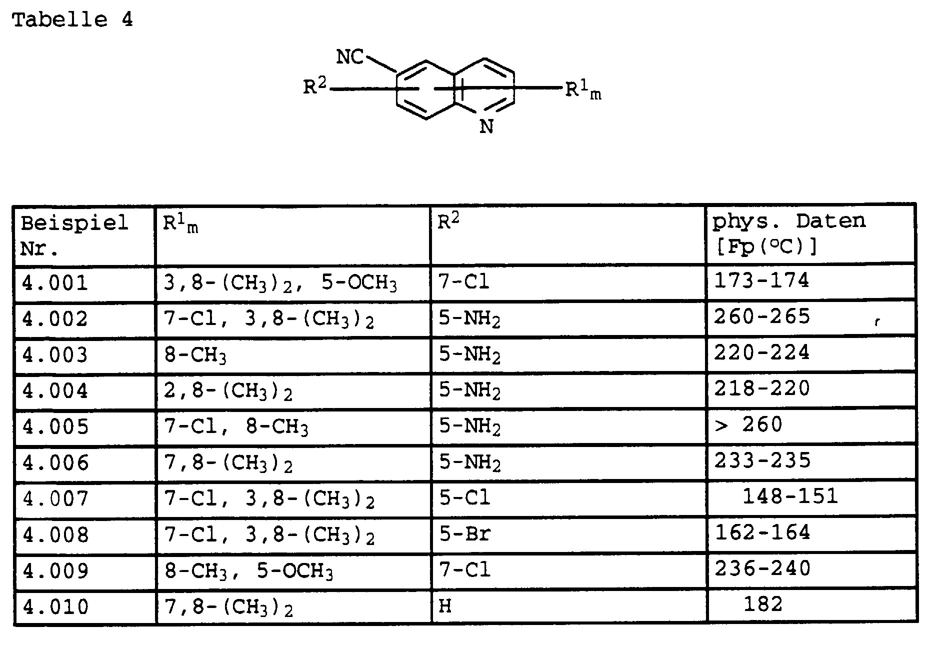

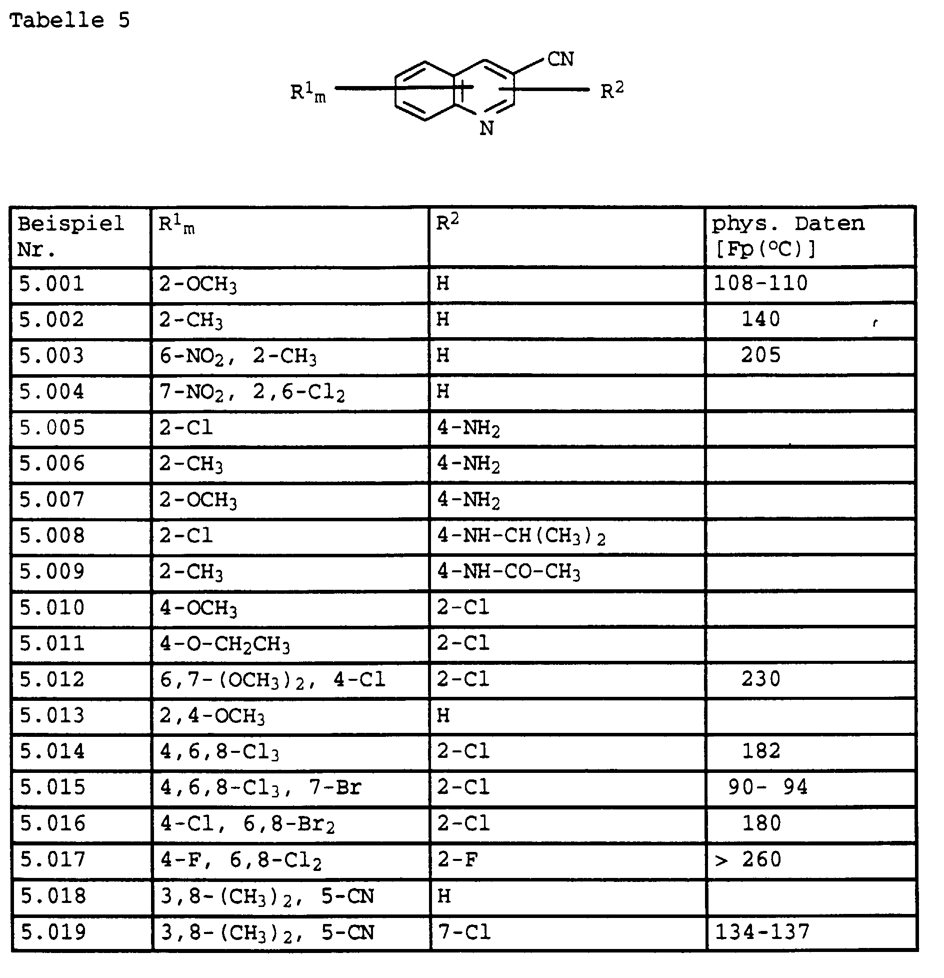

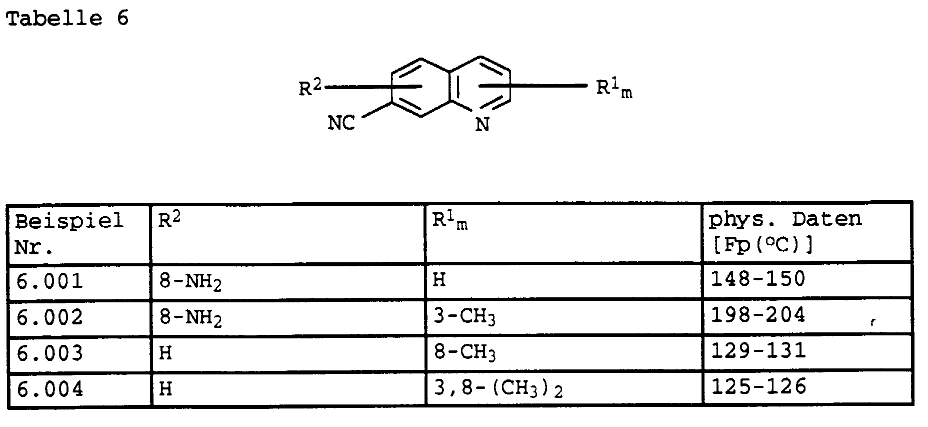

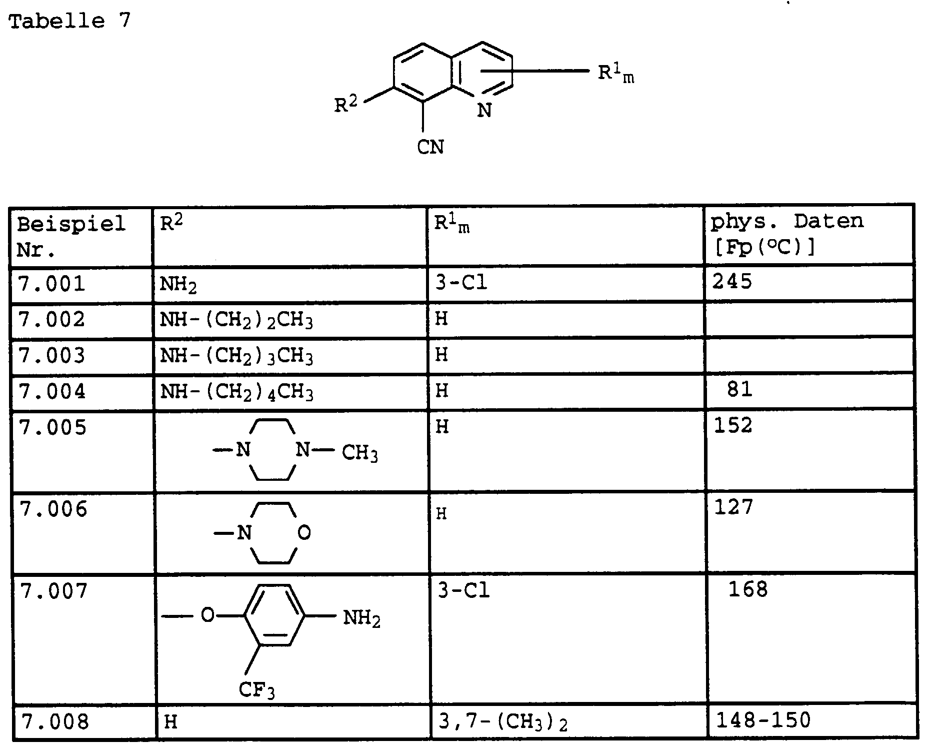

In der folgenden Tabelle 8 sind aus der Literatur bekannte Cyanochinolinverbindungen aufgeführt, welche ebenfalls die Kulturpflanzenverträglichkeit der herbiziden Wirkstoffe der Formeln IX und X zu steigern vermögen.

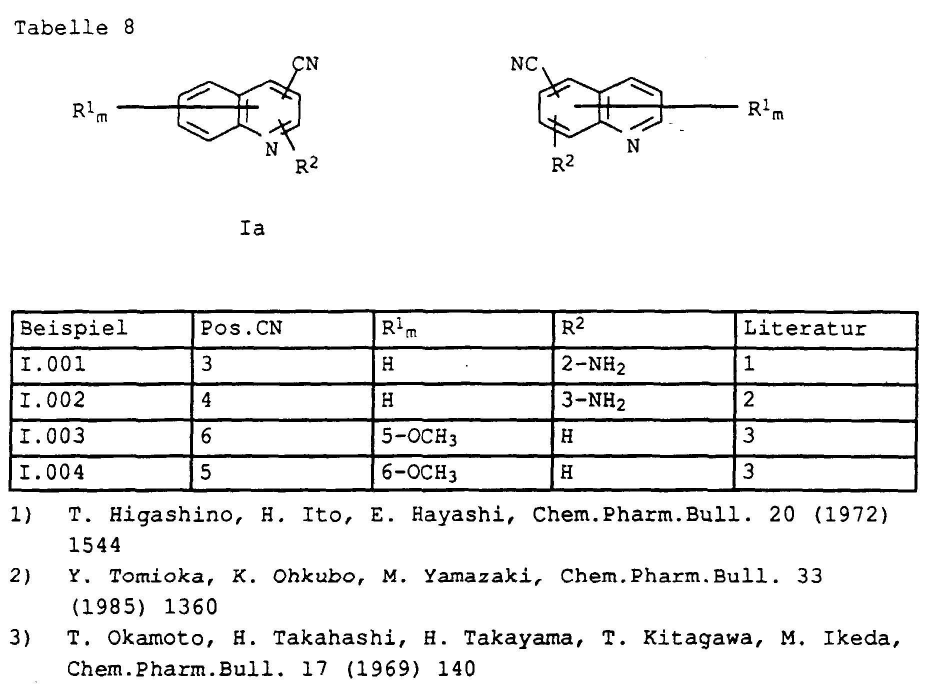

### Beispiele zur biologischen Wirkung

Der Einfluß verschiedener Vertreter der erfindungsgemäßen herbiziden Mittel bzw. Mittelkombinationen, bestehend aus Herbizid und antidotisch wirkender Verbindung, auf das Wachstum von erwünschten und unerwünschten Pflanzen im Vergleich zum herbiziden Wirkstoff allein wird durch die folgenden biologischen Beispiele aus Gewächshausversuchen belegt:

Als Kulturgefäße dienten Plastikblumentöpfe mit rund 300 cm³ Inhalt und lehmiger Sand mit etwa 3,0 % Humus als Substrat. Die Samen der Testpflanzen wurden nach Arten getrennt, flach eingesät und befeuchtet. Danach wurden die Gefäße mit durchsichtigen Plastikhauben abgedeckt, bis die Samen gleichmäßig gekeimt und die Pflanzen angewachsen waren.

Für die Nachauflaufbehandlung wurden die Testpflanzen je nach Wuchsform erst bis zu einer Wuchshöhe von 3 bis 20 cm angezüchtet und erst dann behandelt. Die herbiziden Mittel wurden hierbei in Wasser als Verteilungsmittel suspendiert oder emulgiert und mittels fein verteilender Düsen gespritzt.

Die Versuchsgefäße wurden im Gewächshaus aufgestellt, wobei für wärmeliebende Arten 18 bis 30°C und für solche gemäßigter Klimate 10 bis 25°C bevorzugt wurden.

Die Versuchsperiode erstreckte sich über 3 bis 5 Wochen. Während dieser Zeit wurden die Pflanzen gepflegt, und ihre Reaktion auf die einzelnen Behandlungen wurde erfaßt.

Bewertet wurde die Schädigung der Testpflanzen anhand einer Skala von 0 bis 100 % im Vergleich zu den unbehandelten Kontrollpflanzen. Dabei bedeutet 0 keine Schädigung und 100 eine völlige Zerstörung der Pflanzen.

Die in den Gewächshausversuchen verwendeten Pflanzen setzten sich aus folgenden Arten zusammen:

| Lat. Name | Deutscher Name |
|---|---|
| Brachiaria platyphylla | |
| Lolium multiflorum | Ital. Raygras |
| Oryza sativa | Reis |
| Setaria faberii | Borstenhirse |
| Setaria italica | Kolbenhirze |
| Triticum aestivum | Sommerweizen |
| Zea mays | Mais |

Als Beispielsherbizid der Cyclohexenonderivate der Formel X diente

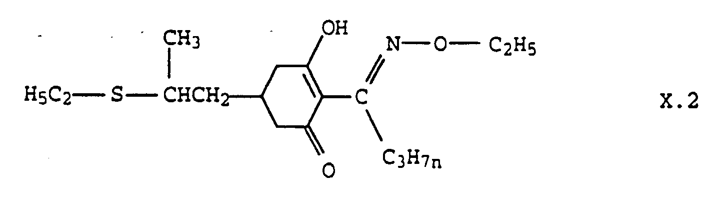

Der herbizide Wirkstoff X.2 wurde als kommerziell formuliertes Produkt (184 g/l EC auch allein jeweils unter Zugabe von derjenigen Menge an Lösungsmittelsystem XXII - Leerformulierung -) in die Spritzbrühe gegeben und gemeinsam mit der antidotischen Verbindung ausgebracht.

Als weiteres Beispiel der herbiziden Cyclohexenonderivate der Formel X wurde 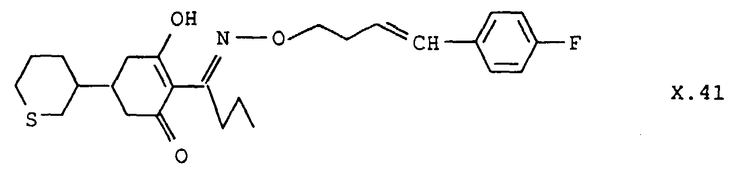 eingesetzt.

Der Wirkstoff X.41 wurde als Emulsionskonzentrat mit 200 g Wirkstoff/1 ebenfalls allein unter Zugabe der für die Antidots nötigen Lösungsmittelmengen, 80 % Cyclohexanon und 20 % Emulphor EL mit 10 % (Gew.-%) Wirkstoff, appliziert.

Sämtliche antidotisch wirkende Verbindungen wurden für die Nachauflaufbehandlung in einem Gemisch, bestehend aus 80 % Cyclohexanon und 20 % Emulphor EL mit 10 % (Gew.-%) Wirkstoff aufbereitet.

Die anschließenden Tabellen dokumentieren die antidotische Wirkung der erfindungsgemäßen Verbindungen 1.010, 3.003, 4.002, 4.003, 5.007, 6.002 und 7.007.

Dabei verbessern diese Verbindungen deutlich die Verträglichkeit des Wirkstoffs X.2 sowie auch des Wirkstoffs X.41 für zu der Familie der Gramineen (Gräser) zählende Kulturpflanzen.

**Tabelle 9**

| Verbesserung der Verträglichkeit des herbiziden Wirkstoffs X.2 für Kulturpflanzen durch Kombination mit der Verbindung 1.010 bei Nachauflaufanwendung im Gewächshaus | | | | |
|---|---|---|---|---|
| Aufwandmenge | | Testpflanzen und Schädigung [%] | | |
| [kg/ha a.S.] | | Kulturpflanze | unerw.Pflanzen | |
| herbizider Wirkstoff X.2 | Antidot 1.010 | Triticum aestivum¹⁾ | Lolium multiflorum | Setaria italica |
| 0.06 | - | 35 | 100 | 90 |
| 0.06 | 0.25 | 10 | 100 | 90 |

| | | | | |
|---|---|---|---|---|
| ¹⁾ cv. "Star" | | | | |

**Tabelle 10**

| Verbesserung der Verträglichkeit des herbiziden Wirkstoffs X.2 für Kulturpflanzen durch Kombination mit der Verbindung 3.003 bei Nachauflaufanwendung im Gewächshaus | | | | | |
|---|---|---|---|---|---|
| Aufwandmenge | | Testpflanzen und Schädigung [%] | | | |
| [kg/ha a.S.] | | Kulturpflanzen | | unerw.Pflanzen | |
| herbizider Wirkstoff X.2 | Antidot 3.003 | Oryza sativa¹⁾ | Triticum aestivum²⁾ | Lolium multiflorum | Setaria italica |
| 0.03 | - | 50 | 30 | 100 | 98 |
| 0.03 | 0.125 | 10 | 10 | 100 | 95 |

| | | | | | |
|---|---|---|---|---|---|
| ¹⁾ cv. "Bahia" | | | | | |
| ²⁾ cv. "Star" | | | | | |

**Tabelle 11**

| Verbesserung der Verträglichkeit des herbiziden Wirkstoffs X.2 für Kulturpflanzen durch Kombination mit der Verbindung 4.003 bei Nachauflaufanwendung im Gewächshaus | | | | | |
|---|---|---|---|---|---|
| Aufwandmenge | | Testpflanzen und Schädigung [%] | | | |
| [kg/ha a.S.] | | Kulturpflanzen | | unerw.Pflanzen | |
| herbizider Wirkstoff X.2 | Antidot 4.003 | Oryza sativa¹⁾ | Brachiaria plat. | Lolium multiflorum | Setaria italica |
| 0.03 | - | 50 | 85 | 100 | 98 |
| 0.03 | 0.125 | 15 | 80 | 100 | 98 |

| | | | | | |
|---|---|---|---|---|---|
| ¹⁾ cv. "Bahia" | | | | | |

**Tabelle 12**

| Verbesserung der Verträglichkeit des herbiziden Wirkstoffs X.2 für Kulturpflanzen durch Kombination mit der Verbindung 5.007 bei Nachauflaufanwendung im Gewächshaus | | | | |
|---|---|---|---|---|
| Aufwandmenge | | Testpflanzen und Schädigung [%] | | |
| [kg/ha a.S.] | | Kulturpflanze | unerw.Pflanzen | |
| herbizider Wirkstoff X.2 | Antidot 5.007 | Oryza sativa¹⁾ | Brachiaria plat. | Lolium multiflorum |
| 0.06 | - | 90 | 90 | 100 |
| 0.06 | 0.25 | 15 | 100 | 100 |

| | | | | |
|---|---|---|---|---|
| ¹⁾ cv. "Bahia" | | | | |

**Tabelle 13**

| Verbesserung der Verträglichkeit des herbiziden Wirkstoffs X.2 für Kulturpflanzen durch Kombination mit der Verbindung 6.002 bei Nachauflaufanwendung im Gewächshaus | | | | |
|---|---|---|---|---|
| Aufwandmenge | | Testpflanzen und Schädigung [%] | | |
| [kg/ha a.S.] | | Kulturpflanze | unerw.Pflanzen | |
| herbizider Wirkstoff X.2 | Antidot 6.002 | Oryza sativa¹⁾ | Brachiaria plat. | Lolium multiflorum |
| 0.06 | - | 90 | 90 | 100 |
| 0.06 | 0.25 | 15 | 80 | 100 |

| | | | | |
|---|---|---|---|---|
| ¹⁾ cv. "Bahia" | | | | |

**Tabelle 14**

| Verbesserung der Verträglichkeit des herbiziden Wirkstoffs X.2 für Kulturpflanzen durch Kombination mit der Verbindung 7.007 bei Nachauflaufanwendung im Gewächshaus | | | |
|---|---|---|---|
| Aufwandmenge | | Testpflanzen und Schädigung [%] | |
| [kg/ha a.S.] | | Kulturpflanze | unerw.Pflanze |
| herbizider Wirkstoff X.2 | Antidot 7.007 | Oryza sativa¹⁾ | Setaria italica |
| 0.06 | - | 50 | 98 |
| 0.06 | 0.125 | 0 | 95 |

| | | | |
|---|---|---|---|
| ¹⁾ cv. "Bahia" | | | |

**Tabelle 15**

| Verbesserung der Verträglichkeit des herbiziden Wirkstoffs X.2 für Kulturpflanzen durch Kombination mit der Verbindung 7.007 bei Nachauflaufanwendung im Gewächshaus | | | | |
|---|---|---|---|---|
| Aufwandmenge | | Testpflanzen und Schädigung [%] | | |
| [kg/ha a.S.] | | Kulturpflanze | unerw.Pflanzen | |
| herbizider Wirkstoff X.2 | Antidot 7.007 | Zea mays¹⁾ | Lolium multiflorum | Setaria italica |
| 0.06 | - | 100 | 100 | 100 |
| 0.06 | 0.25 | 20 | 100 | 95 |

| | | | | |
|---|---|---|---|---|
| ¹⁾ cv. "Mutin" | | | | |

**Tabelle 16**

| Verbesserung der Verträglichkeit des herbiziden Wirkstoffs X.41 für Kulturpflanzen durch Kombination mit der Verbindung 4.002 bei Nachauflaufanwendung im Gewächshaus | | | | |
|---|---|---|---|---|
| Aufwandmenge | | Testpflanzen und Schädigung [%] | | |
| [kg/ha a.S.] | | Kulturpflanze | unerw.Pflanzen | |
| herbizider Wirkstoff X.41 | Antidot 4.002 | Zea mays | Setaria faberii | Setaria italica |
| 0.125 | - | 40 | 90 | 85 |
| 0.125 | 0.5 | 10 | 80 | 80 |

## Patentansprüche

1. Herbizides Mittel, enthaltend mindestens eine Cyanochinolinverbindung der allgemeinen Formel Ia oder Ib 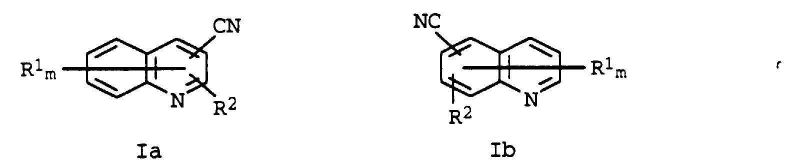 in der die Substituenten und der Index die folgende Bedeutung haben:
R¹ Cyano, Halogen, C₁-C₄-Alkyl, C₁-C₄-Alkoxy oder Phenoxy, wobei der Phenylring ein bis drei der folgenden Reste tragen kann: Halogen, C₁-C₄-Alkyl, C₁-C₄-Halogenalkyl, C₁-C₄-Alkoxy, C₁-C₄-Alkylthio, C₂-C₄-Alkenyl, Nitro, Amino, Nitro-C₂-C₄-alkenyl und/oder Benzyl;
m 0, 1, 2 oder 3, wobei die Reste R¹ verschieden sein können, wenn m 2 oder 3 bedeutet;
R² Wasserstoff, Halogen oder eine Gruppe NR³R⁴;
R³ Wasserstoff oder eine C₁-C₄-Alkylgruppe, welche ein bis fünf Halogenatome und/oder eine der folgenden Gruppen tragen kann: Hydroxy, C₁-C₄-Alkoxy, C₁-C₄-Halogenalkoxy, C₁-C₄-Alkylthio, Amino, C₁-C₄-Alkylamio oder Di-C₁-C₄-alkylamio;
R⁴ eine der bei R³ genannten Gruppen; eine C₁-C₄-Alkylamiogruppe; eine Di-C₁-C₄-alkylamiogruppe; eine C₁-C₄-Alkylgruppe, welche einen der folgenden Reste trägt: C₁-C₄-Alkoxycarbonyl, 1-Pyrrolidinyl oder 1-Imidazolyl;
oder
R³ und R⁴ gemeinsam eine C₄-C₅-Alkylenkette, welche durch ein Sauerstoff- oder ein Stickstoff-Glied unterbrochen sein kann, wobei das Stickstoffatom seinerseits eine C₁-C₄-Alkylgruppe oder eine Amino-C₁-C₄-alkylgruppe tragen kann
oder eine 1,3-Butadienylkette, welche ein oder zwei der bei R¹ genannten Reste oder eine Formylgruppe tragen kann,
sowie landwirtschaftlich brauchbare Salze der Verbindungen Ia und Ib, sofern diese Verbindungen einen basischen Stickstoffsubstituenten oder einen sauren Hydroxysubstituenten enthalten
und mindestens einen herbiziden Wirkstoff aus der Gruppe der Cyclohexenonoximether der Formel X 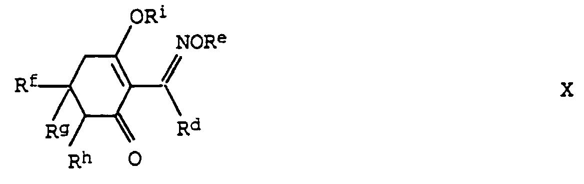 enthalten, in welcher die Substituenten folgende Bedeutung haben:
R^{d} eine C₁-C₄-Alkylgruppe;
R^{e} eine C₁-C₄-Alkylgruppe, eine C₃-C₄-Alkenylgruppe, eine C₃-C₄-Alkinylgruppe, eine C₃-C₄-Halogenalkylengruppe oder eine Thenylgruppe, welche durch ein Halogenatom substituiert sein kann; eine C₃-C₄-Alkenylgruppe, welche einen Phenylrest trägt, wobei dieser ein bis drei der folgenden Reste tragen kann: Halogen, C₁-C₄-Alkyl, C₁-C₄-Akoxy, C₁-C₄-Halogenalkyl und/oder C₁-C₄-Halogenalkoxy;
R^{f} eine C₁-C₄-ASky-gruppe, welche ein- oder zweifach durch C₁-C₄-Alkylthio oder C₁-C₄-Alkoxy substituiert sein kann;
ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, und wobei dieser Ring bis zu drei der folgenden Reste tragen kann: Hydroxy, Halogen,
C₁-C₄-Alkyl, C₁-C₄-Halogenalkyl, C₁-C₄-Alkoxy und/oder C₁-C₄-Alkylthio;
ein 10-gliedriger gesättigter oder einfach ungesättigter Heterocyclus, welcher zwei Sauerstoffatome oder Schwefelatome enthält und durch bis zu drei C₁-C₄-Alkylgruppen und/oder Methoxygruppen substituiert sein kann;
ein Phenylrest, ein Pyridylrest, ein Thiazolylrest, ein Pyrazolylrest, ein Pyrrolylrest oder ein Isoxazolylrest, wobei diese Reste bis zu drei der folgenden Gruppen tragen können: C₁-C₄-Alkyl, C₁-C₄-Halogenalkyl, C₁-C₄-Alkoxy, C₁-C₄-Alkylthio, C₃-C₆-Alkenyloxy, C₃-C₆-Alkinyloxy, C₁-C₄-Alkoxy-C₁-C₃-alkyl, C₁-C₄-Dialkoxy-C₁-C₃-alkyl, Formyl, Halogen und/oder Benzoylamino
R^{g} Wasserstoff, Hydroxy oder, wenn R^{f} die Bedeutung C₁-C₆-Alkylgruppe hat, eine C₁-C₆-Alkylgruppe;
R^{h} Wasserstoff, eine Cyanogruppe, ein Halogenatom, eine C₁-C₄-Alkoxycarbonylgruppe oder eine C₁-C₄-Alkylketoximgruppe und
Rⁱ Wasserstoff oder das Äquivalent eines umweltverträglichen Kations.

2. Herbizides Mittel nach Anspruch 1, enthaltend 2,3-Dicyanochinolin, 6-(N-2-Aminoethyl)-amino-5-cyano-chinolin, 5-Amino-7-chloro-6-cyano-3,8-dimethyl-chinolin, 5-Amino-6-cyano-8-methyl-chinolin, 4-Amino-3-cyano-2-methoxy-chinolin, 8-Amino-7-cyano-3-methyl-chinolin oder 7-[(4-Amino-2-trifluormethyl)-phenoxy]-3-chloro-8-cyano-chinolin.

3. Herbizides Mittel nach Anspruch 1 oder 2, dadurch gekennzeichnet, daß das Gewichtsverhältnis Cyanochinolinverbindung Ia oder Ib zu herbizidem Cyclohexenonoximether X 0.01:1 bis 10:1 Gew.-Teile beträgt.

4. Verfahren zur selektiven Bekämpfung von unerwünschtem Pflanzenwuchs, dadurch gekennzeichnet, daß man ein Cyanochinolin der Formel Ia oder Ib gemäß Anspruch 1 und ein Cyclohexenonderivat der Formel X gemäß Anspruch 1 vor, bei oder nach der Aussaat der Kulturpflanzen, vor oder während des Auflaufens der Kulturpflanzen gleichzeitig oder nacheinander ausbringt.

5. Verfahren zur selektiven Bekämpfung von unerwünschtem Pflanzenwuchs, dadurch gekennzeichnet, daß man ein Cyanochinolin gemäß Anspruch 2 und ein Cyclohexenonderivat der Formel X gemäß Anspruch 1 vor, bei oder nach der Aussaat der Kulturpflanzen, vor oder während des Auflaufens der Kulturpflanzen gleichzeitig oder nacheinander ausbringt.

6. Verfahren zur Verhinderung von Schädigungen von Kulturpflanzen durch herbizide Cyclohexenonderivateder Formel X gemäß Anspruch 1, dadurch gekennzeichnet, daß man das Saatgut der Kulturpflanzen mit einer antagonistisch wirksamen Menge eines Cyanochinolins der Formel Ia oder Ib gemäß Anspruch 1 behandelt.

7. Verfahren zur Verhinderung von Schädigungen von Kulturpflanzen durch herbizide Cyclohexenonderivate der Formel X gemäß Anspruch 1, dadurch gekennzeichnet, daß man das Saatgut der Kulturpflanzen mit einer antagonistisch wirksamen Menge eines Cyanochinolins gemäß Anspruch 2 behandelt.

## Claims

1. A herbicide containing one or more cyanoquinoline compounds of the formula Ia or Ib 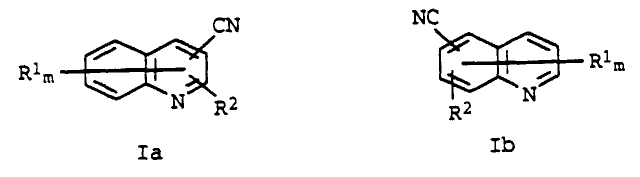 where
R¹ is cyano, halogen, C₁-C₄-alkyl, C₁-C₄-alkoxy or phenoxy, where the phenyl ring may carry from one to three of the following radicals: halogen, C₁-C₄-alkyl, C₁-C₄-haloalkyl, C₁-C₄-alkoxy, C₁-C₄-alkylthio, C₂-C₄-alkenyl, nitro, amino, nitro-C₂-C₄-alkenyl and/or benzyl;
m is 0, 1, 2 or 3, and the radicals R¹ may be different when m is 2 or 3;
R² is hydrogen, halogen or NR³R⁴;
R³ is hydrogen or C₁-C₄-alkyl which may carry from one to five halogen atoms and/or one of the following groups: hydroxyl, C₁-C₄-alkoxy, C₁-C₄-haloalkoxy, C₁-C₄-alkylthio, amino, C₁-C₄-alkylamino or di-C₁-C₄-alkylamino;
R⁴ is one of the groups stated for R³; C₁-C₄-alkylamino; di-C₁-C₄-alkylamino; C₁-C₄-alkyl which carries one of the following radicals: C₁-C₄-alkoxycarbonyl, 1-pyrrolidinyl or 1-imidazolyl;
or R³ and R⁴ together form a C₄- or C₅-alkylene chain which may be interrupted by an oxygen or nitrogen member, where the nitrogen atom in turn may carry a C₁-C₄-alkyl or amino-C₁-C₄-alkyl group,
or a 1,3-butadienyl chain which may carry one or two of the radicals stated for R¹ or formyl,
and agriculturally usable salts of the compounds Ia and Ib, provided that these compounds contain a basic nitrogen substituent or an acidic hydroxyl substituent,
and one or more herbicidal active ingredients from the group consisting of the cyclohexenone oxime ethers of the formula X 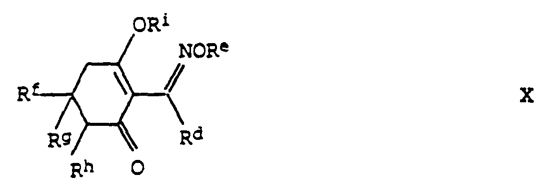
where
R^{d} is C₁-C₄-alkyl;
R^{e} is C₁-C₄-alkyl, C₃- or C₄-alkenyl, C₃- or C₄-alkynyl, C₃- or C₄-haloalkylene or thenyl, which may be substituted by a halogen atom; C₃- or C₄-alkenyl which carries a phenyl radical which may carry from one to three of the following radicals: halogen, C₁-C₄-alkyl, C₁-C₄-alkoxy, C₁-C₄-haloalkyl and/or C₁-C₄-haloalkoxy;
R^{f} is C₁-C₄-alkyl which may be monosubstituted or disubstituted by C₁-C₄-alkylthio or C₁-C₄-alkoxy;
a 5-membered or 6-membered saturated or monounsaturated ring system which, in addition to carbon members, may contain an oxygen or sulfur atom or a sulfoxyl or sulfonyl group, and this ring may carry up to three of the following radicals: hydroxyl, halogen, C₁-C₄-alkyl, C₁-C₄-haloalkyl, C₁-C₄-alkoxy and/or C₁-C₄-alkylthio;
a 10-membered saturated or monounsaturated heterocyclic structure which contains two oxygen or sulfur atoms and may be substituted by up to three C₁-C₄-alkyl groups and/or methoxy groups;
a phenyl, pyridyl, thiazolyl, pyrazolyl, pyrrolyl or isoxazolyl radical which may carry up to three of the following groups: C₁-C₄-alkyl, C₁-C₄-haloalkyl, C₁-C₄-alkoxy, C₁-C₄-alkylthio, C₃-C₆-alkenyloxy, C₃-C₆-alkynyloxy, C₁-C₄-alkoxy-C₁-C₃-alkyl, C₁-C₄-dialkoxy-C₁-C₃-alkyl, formyl, halogen and/or benzoylamino;
R^{g} is hydrogen, hydroxyl or, if R^{f} is C₁-C₆-alkyl, a C₁-C₆-alkyl group;
R^{h} is hydrogen, cyano, halogen, C₁-C₄-alkoxycarbonyl or C₁-C₄-alkylketoxime and
Rⁱ is hydrogen or one equivalent of an environmentally compatible cation.

2. A herbicide as claimed in claim 1, containing 2,3-dicyanoquinoline, 6-N-(2-aminoethyl)-amino-5-cyanoquinoline, 5-amino-7-chloro-6-cyano-3,8-dimethylquinoline, 5-amino-6-cyano-8-methylquinoline, 4-amino-3-cyano-2-methoxyquinoline, 8-amino-7-cyano-3-methylquinoline or 7-[(4-amino-2-trifluoromethyl)-phenoxy]-3-chloro-8-cyanoquinoline.

3. A herbicide as claimed in claim 1 or 2, wherein the weight ratio of cyanoquinoline compound Ia or Ib to herbicidal cyclohexenone oxime ether is from 0.01 : 1 to 10 : 1.

4. A method for selectively controlling undesirable plant growth, wherein a cyanoquinoline of the formula Ia or Ib as claimed in claim 1 and a cyclohexenone derivative of the formula X as claimed in claim 1 are applied simultaneously or in succession before, during or after sowing of the crops or before or during emergence of the crops.

5. A method for selectively controlling undesirable plant growth, wherein a cyanoquinoline as claimed in claim 2 and a cyclohexenone derivative of the formula X as claimed in claim 1 are applied simultaneously or in succession before, during or after sowing of the crops or before or during emergence of the crops.

6. A method for preventing damage to crops by herbicidal cyclohexenone derivatives of the formula X as claimed in claim 1, wherein the seed of the crops is treated with an antagonistic amount of a cyanoquinoline of the formula Ia or Ib as claimed in claim 1.

7. A method for preventing damage to crops by herbicidal cyclohexenone derivatives of the formula X as claimed in claim 1, wherein the seed of the crops is treated with an antagonistic amount of a cyanoquinoline as claimed in claim 2.

## Revendications

1. Agent herbicide, contenant au moins un composé de cyanoquinoléine de formule générale Ia ou Ib 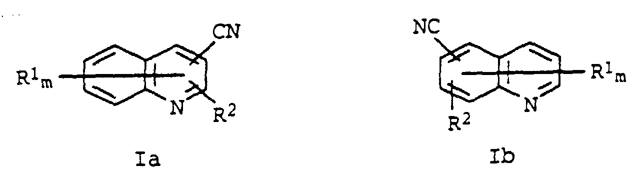 où les substituants et l'indice ont la signification suivante:
R¹ un groupe cyano, halogène, alkyle en C₁-C₄, alcoxy en C₁-C₄ ou phénoxy, tandis que le cycle phényle peut porter un à trois des restes suivants: halogène, alkyle en C₁-C₄, halogénoalkyle en C₁-C₄, alcoxy en Cₗ-C₄, alkylthio en C₁-C₄, alcényle en C₂-C₄, nitro, amino, nitro-alcényle en C₂-C₄ et/ou benzyle;
m vaut 0, 1, 2 ou 3, tandis que les restes R¹ peuvent être différents lorsque m vaut 2 ou 3;
R² hydrogène, halogène ou un groupe NR³R⁴;
R³ hydrogène ou un groupe alkyle en C₁-C₄, qui peut porter un à cinq atomes d'halogène et/ou l'un des groupes suivants : hydroxy, alcoxy en C₁-C₄, halogénoalcoxy en C₁-C₄, alkylthio en C₁-C₄, amino, alkylamino en C₁-C₄ ou di-alkylamino en C₁-C₄;
R⁴ l'un des groupes cités pour R³; un groupe alkylamino en C₁-C₄; un groupe di-alkylamino en C₁-C₄; un groupe alkyle en C₁-C₄, qui porte l'un des restes suivants: alcoxy(C₁-C₄)carbonyle, 1-pyrrolidinyle ou 1-imidazolyle;
ou
R³ et R⁴ représentent ensemble une chaîne alkylène en C₄-C₅, qui peut être interrompue par un chaînon oxygène ou un chaînon azote, tandis que l'atome d'azote peut à son tour porter un groupe alkyle en C₁-C₄ ou un groupe aminoalkyle en C₁-C₄
ou une chaîne 1,3-butadiényle, qui peut porter un ou deux des restes cités pour R³ ou un groupe formyle,
ainsi que les sels utilisables en agriculture des composés Ia et Ib, dans la mesure où ces composés contiennent un substituant azoté basique ou un substituant hydroxylé acide
et au moins une substance à activité herbicide choisie dans le groupe des cyclohexénone-oxime-éthers de formule X 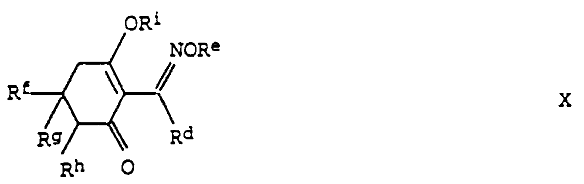 où les substituants ont la signification suivante:
R^{d} un groupe alkyle en C₁-C₄;
R^{e} un groupe alkyle en C₁-C₄, un groupe alcényle en C₃-C₄, un groupe alcynyle en C₃-C₄, un groupe halogénoalkylène en C₃-C₄ ou un groupe thiényle, qui peut être substitué par un atome d'halogène; un groupe alcényle en C₃-C₄, qui porte un reste phényle, tandis que celui-ci peut porter un à trois des restes suivants: halogène, alkyle en C₁-C₄, alcoxy en C₁-C₄, halogénoalkyle en C₁-C₄ et/ou halogénoalcoxy en C₁-C₄;
R^{f} un groupe alkyle en C₁-C₄, qui peut être substitué une ou deux fois par un groupe alkylthio en C₁-C₄ ou alcoxy en C₁-C₄;
un système cyclique à 5 ou 6 chaînons, saturé ou portant une insaturation, qui peut contenir, outre les chaînons hydrocarbonés, un atome d'oxygène, un atome de soufre, un groupe sulfoxyde ou un groupe sulfone, et où ce cycle peut porter jusqu'à trois des restes suivants: hydroxy, halogène, alkyle en C₁-C₄, halogénoalkyle en C₁-C₄, alcoxy en C₁-C₄ et/ou alkylthio en C₁-C₄;
un hétérocycle à 10 chaînons, saturé ou portant une insaturation, qui contient deux atomes d'oxygène ou deux atomes de soufre et peut être substitué par jusqu'à trois groupes alkyle en C₁-C₄ et/ou groupes méthoxy;
un reste phényle, un reste pyridyle, un reste thiazolyle, un reste pyrazolyle, un reste pyrrolyle ou un reste isoxazolyle, tandis que ces restes peuvent porter jusqu'à trois des groupes suivants: alkyle en C₁-C₄, halogénoalkyle en C₁-C₄, alcoxy en C₁-C₄, alkylthio en C₁-C₄, alcényloxy en C₃-C₆, alcynyloxy en C₃-C₆, alcoxy(C₁-C₄)-alkyle(C₁-C₃), dialcoxy(C₁-C₄)-alkyle(C₁-C₃), formyle, halogène et/ou benzoylamino;
R^{g} hydrogène, hydroxy ou, lorsque R^{f} représente un groupe alkyle en C₁-C₆, un groupe alkyle en C₁-C₆;
R^{h} hydrogène, un groupe cyano, un atome d'halogène, un groupe alcoxy(C₁-C₄)carbonyle ou un groupe alkyl(C₁-C₄)cétoxime et
Rⁱ hydrogène ou l'équivalent d'un cation acceptable pour l'environnement.

2. Agent herbicide selon la revendication 1, contenant de la 2,3-dicyano-quinoléine, de la 6-(N-2-aminoéthyl)-amino-5-cyano-quinoléine, de la 5-amino-7-chloro-6-cyano-3,8-diméthyl-quinoléine, de la 5-amino-6-cyano-8-méthyl-quinoléine, de la 4-amino-3-cyano-2-méthoxy-quinoléine, de la 8-amino-7-cyano-3-méthyl-quinoléine ou de la 7-[(4-amino-2-trifluorométhyl)-phénoxy]-3-chloro-8-cyano-quinoléine.

3. Agent herbicide selon l'une des revendications 1 ou 2, caractérisé par le fait que le rapport pondéral du composé de cyanoquinoléine Ia ou Ib au cyclohexénoneoxime-éther herbicide X s'élève à 0,01:1 à 10:1 parties en poids.

4. Procédé pour la lutte sélective contre la croissance indésirable des plantes, caractérisé par le fait qu'on met en oeuvre simultanément ou successivement une cyanoquinoléine de formule Ia ou Ib selon la revendication 1 et un dérivé de cyclohexénone de formule X selon la revendication 1, avant, pendant ou après le semis des plantes cultivées, avant ou pendant la levée des plantes cultivées.

5. Procédé pour la lutte sélective contre la croissance indésirable des plantes, caractérisé par le fait qu'on met en oeuvre une cyanoquinoléine selon la revendication 2 et un dérivé de cyclohexénone de formule X selon la revendication 1, simultanément ou successivement, avant, pendant ou après le semis des plantes cultivées, avant ou pendant la levée des plantes cultivées.

6. Procédé pour l'inhibition des dommages aux plantes cultivées par des dérivés de cyclohexénone herbicides de formule X selon la revendication 1, caractérisé par le fait qu'on traite les semences des plantes cultivées avec une quantité à activité antagoniste d'une cyanoquinoléine de formule Ia ou Ib selon la revendication 1.

7. Procédé pour l'inhibition des dommages aux plantes cultivées par des dérivés de cyclohexénone herbicides de formule X selon la revendication 1, caractérisé par le fait qu'on traite les semences des plantes cultivées avec une quantité à activité antagoniste d'une cyanoquinoléine selon la revendication 2.
